# EUROPEAN PATENT SPECIFICATION

(11) **EP 2 842 639 B1**
(45) Date of publication and mention of the grant of the patent: **25.07.2018**
(21) Application number: 13781096.6
(22) Date of filing: 26.04.2013
(51) Int. Cl.: B05B 17/06, A01M 1/20, A61L 2/18, A61L 2/22, A61L 9/14, F24F 6/12, B05B 17/00

(54) **ULTRASONIC ATOMIZATION DEVICE**
ULTRASCHALLZERSTÄUBER
DISPOSITIF D'ATOMISATION À ULTRASONS

(30) Priority: 27.04.2012 JP 2012103620
(43) Date of publication of application: 04.03.2015
(73) Proprietor: Sumitomo Chemical Company, Limited, Tokyo 104-8260 (JP)
(72) Inventor: KAWANO, Hiroyuki, Takarazuka-shi Hyogo 665-8555 (JP); HARADA, Tetsuo, Takarazuka-shi Hyogo 665-8555 (JP); TAKAHATA, Daisuke, Ageo-shi Saitama 362-8561 (JP); UEDA, Kazuyuki, Ageo-shi Saitama 362-8561 (JP)
(74) Representative: Vossius & Partner Patentanwälte Rechtsanwälte mbB
(86) International application number: PCT/JP2013/062344
(87) International publication number: WO 2013/161985

(56) References cited:
- JP-A- S58 216 753
- JP-A- 2002 536 173
- JP-A- 2012 115 828
- US-A1- 2009 242 663
- US-A1- 2009 242 663
- US-A1- 2011 315 786
- US-B1- 6 378 780
- US-B1- 6 386 462

## Description

### Technical Field

The present invention relates to an ultrasonic atomizing device for atomizing liquid such as water or a solution by ultrasonic vibration.

### Background Art

An ultrasonic atomizing device has been known as means for atomizing, in an interior or exterior space, liquid such as a solution containing an active ingredient. The ultrasonic atomizing device includes (i) a piezoelectric element which generates ultrasonic vibration when supplied with electricity and (ii) a vibrating plate which has many micropores and is attached to the piezoelectric element. The ultrasonic atomizing device is configured to atomize liquid by (i) supplying the liquid to the micropores and (ii) causing ultrasonic vibration on the vibrating plate by vibration of the piezoelectric element.

An atomizing device of Patent Literature 1 includes a pressurizing chamber for containing a liquid, a nozzle plate having a plurality of nozzles and facing the pressurizing chamber, and an electric vibrator for urging the nozzle plate and exciting flexural vibration, and a protrusion section is provided in the nozzle plate and micropores are provided in the protrusion section. The micropores directly contact with a liquid and are directed in a horizontal direction (a direction vertical to a direction of gravity). With this, the atomizing device of Patent Literature 1 atomizes a liquid in the horizontal direction.

An atomizer of Patent Literature 2 includes a film capable of vibrating for atomizing a liquid and a vibration section for vibrating the film. The film has a first curving portion and a second curving portion having a different curvature from that of the first curving portion, and atomizes a liquid in contact with the film by vibration of the vibration section. The atomizer of Patent Literature 2 atomizes a liquid downward (in a direction of gravity).

In an ultrasonic wave atomizing device of Patent Literature 3, a piezoelectric element is vibrated by a complex constituting of the piezoelectric element and a vibrator is driven and such vibration is propagated to the vibrator. A liquid supplied to a bottom surface of a vibrator in contact with a liquid holding agent is atomized through pores provided in the vibrator in accordance with the vibration of the vibrator.

Patent Literature 4 relates to a medical liquid droplet apparatus, comprising: a driving base, for providing a vibrating energy; and a polymeric film, having a plurality of tiny openings formed thereon and being disposed on the driving base for receiving the vibrating energy so as to generating liquid droplets.

Patent Literature 5 relates to an atomizing unit comprising: a piezoelectric vibrator including a vibrating film with through holes in an atomization region in a center of the vibrating film, and a piezoelectric body on which the vibrating film is mounted, the piezoelectric body being configured to cause the vibrating film to vibrate; an elastic film disposed to face the atomization region; and a liquid feeder supported by the elastic film so as to face the atomization region of the vibrating film with a gap interposed therebetween, the liquid feeder being configured to feed a liquid to the atomization region.

### Citation List

### Patent Literatures

Patent Literature 1
   Japanese Patent Application Publication, *Tokukaisho,* No. 58-216753 (Publication date: December 16, 1983)
Patent Literature 2
   U.S. Pat. No. 7472701 (Registration date: January 6, 2009)
Patent Literature 3
   Japanese Patent Application Publication, *Tokukaihei,* No. 6-7721 (Publication date: January 18, 1994)
Patent Literature 4
   US 2009/0242663 A1
Patent Literature 5
   US 2011/0315786 A1

### Summary of Invention

### Technical Problem

However, techniques of Patent Literatures 1 to 3 have the following problems.

Specifically, a liquid directly contacts with a vibrating plate in the atomizing device of Patent Literature 1 and in the atomizer of Patent Literature 2, and therefore, if an atomizing opening is provided upward, the liquid becomes further from the vibrating plate as a residual liquid is reduced, and consequently atomization of the liquid to an outside of the device is hindered. In addition, when the residual liquid is reduced, air bubbles increase between the vibrating plate and a surface of the liquid, and consequently the atomization of the liquid to the outside of the device is hindered.

In the atomizing device of Patent Literature 1, the atomizing opening is directed horizontally, the surface of the liquid becomes lower as the residual liquid is reduced. Therefore, in the atomizing device of the Patent Literature 1, when the surface of the liquid becomes lower than the nozzle, the atomization of the liquid to the outside of the device is hindered.

In the atomizer of Patent Literature 2, the liquid is atomized downward, so that it is possible to atomize a whole amount of the liquid, however, a weight of the liquid is applied as a load to the vibrating plate. Therefore, there is a problem in that the vibrating plate is degraded in a shorter time.

As described above, in both the atomizing device of Patent Literature 1 and the atomizer of Patent Literature 2, it is difficult to atomize the liquid to the outside of the device stably and sustainably.

In the ultrasonic wave atomizing device of Patent Literature 3, a liquid which has been permeated to the liquid holding agent is atomized upward (in a direction opposite from that of gravity) via the vibrator which is in contact with the liquid holding agent. However, in the ultrasonic wave atomizing device of the Patent Literature 3, it is difficult to obtain an enough atomizing height because a center of the vibrator is curved. Therefore, because diffusibility of the liquid to be atomized cannot be satisfactorily obtained, it is difficult to widely spread an effect (sterilization, killing insects, etc.) obtained by atomizing the liquid within a wide range.

The present invention has been made in view of the above problems, and an object of the present invention is to provide an ultrasonic wave atomizing device capable of improving diffusibility of an atomized liquid.

### Solution to Problem

In order to achieve the above object, an ultrasonic wave atomizing device according to the present invention includes:
a liquid absorbent wick (22) for absorbing a liquid from a liquid storage container (20); and
a vibrating plate (32, 50) which has multiple micropores (36) penetrating the vibrating plate (32, 50) in a thickness direction and atomizes the liquid by vibration of a piezoelectric element (31) which generates ultrasonic vibration when supplied with electricity,
   the vibrating plate (32, 50) having a convex part (37) which has a truncated-cone shape and is supplied with the liquid through the liquid absorbent wick (22), wherein the multiple micropores (36) are provided only in an upper base of the convex part (37) of the vibrating plate (32, 50), and
   wherein the vibrating plate (32, 50) is constituted by a thin circular plate made of nickel, nickel alloy, or ferroalloy.

According to the above structure, an ultrasonic wave atomizing device according to the present invention includes a liquid absorbent wick for absorbing a liquid from a liquid storage container. Therefore, by immersing one end of the liquid absorbent wick in a liquid in the liquid storage container and directing the other of the liquid absorbent wick upward (in a direction opposite from that of gravity), it is possible to atomize the liquid upward by vibrating the vibrating plate.

In the ultrasonic wave atomizing device according to the present invention, the vibrating plate has the truncated-cone shaped convex part to which the liquid is supplied through the liquid absorbent wick. Therefore, the ultrasonic wave atomizing device according to the present invention can have a high atomizing height of the liquid, as compared with a case where a liquid is atomized with use of a vibrating plate having a conventional dome shape or the like. Therefore, the ultrasonic wave atomizing device according to the present invention can improve diffusibility of the liquid around the ultrasonic wave atomizing device, and, in a case where the liquid is, for example, an insecticide, it is possible to widely spread an effect of killing insects.

Because the ultrasonic wave atomizing device according to the present invention includes the vibrating plate having the truncated-cone shaped convex part, the ultrasonic wave atomizing device has higher durability than conventional vibrating plates. Therefore, the ultrasonic wave atomizing device according to the present invention can reduce burdens on a user, such as time, effort, and costs for exchanging a vibrating plate.

In order to achieve the above object, an ultrasonic wave atomizing device includes: a liquid absorbent wick for absorbing a liquid to be atomized; a piezoelectric element which has a ring shape when seen from a plan view and generates ultrasonic vibration in a radial direction when supplied with electricity; and a vibrating plate which is fixed to the piezoelectric element so that the vibrating plate may cover a center opening of the piezoelectric element, the vibrating plate having a center being substantially concentric with the center opening of the piezoelectric element, having a truncated-cone-shaped convex part protruding in a direction in which the liquid is atomized, and having multiple micropores in at least an upper base of the truncated-cone-shaped convex part, the multiple micropores penetrating the vibrating plate in a thickness direction, the liquid being supplied to the vibrating plate via the liquid absorbent wick.

According to the above structure, in the ultrasonic wave atomizing device according to the present invention, the vibrating plate has the truncated-cone-shaped convex part to which the liquid is supplied through the liquid absorbent wick. Therefore, the ultrasonic wave atomizing device according to the present invention can have a height at which the liquid is atomized higher, as compared with a case where a liquid is atomized with use of a vibrating plate having a conventional dome shape or the like. Therefore, the ultrasonic wave atomizing device according to the present invention can improve diffusibility of the liquid around the ultrasonic wave atomizing device, and, in a case where the liquid is, for example, an insecticide, it is possible to widely spread an effect of killing insects.

Because the ultrasonic wave atomizing device according to the present invention includes the vibrating plate having the truncated-cone-shaped convex part, the ultrasonic wave atomizing device has higher durability than conventional vibrating plates. Therefore, the ultrasonic wave atomizing device according to the present invention can reduce burdens on a user, such as time, effort, and costs for exchanging a vibrating plate.

### Advantageous Effects of Invention

As described above, an ultrasonic wave atomizing device according to the present invention includes: a liquid absorbent wick for absorbing a liquid from a liquid storage container; and a vibrating plate which has multiple micropores penetrating the vibrating plate in a thickness direction and atomizes the liquid by vibration of a piezoelectric element which generates ultrasonic vibration when supplied with electricity, the vibrating plate having a convex part which has a truncated-cone shape and is supplied with the liquid through the liquid absorbent wick, wherein the multiple micropores are provided only in an upper base of the convex part of the vibrating plate, and wherein the vibrating plate is constituted by a thin circular plate made of nickel, nickel alloy, or ferroalloy.
Further, as described above, an ultrasonic wave atomizing device includes: a liquid absorbent wick for absorbing a liquid to be atomized; a piezoelectric element which has a ring shape when seen from a plan view and generates ultrasonic vibration in a radial direction when supplied with electricity; and a vibrating plate which is fixed to the piezoelectric element so that the vibrating plate may cover a center opening of the piezoelectric element, the vibrating plate having a center being substantially concentric with the center opening of the piezoelectric element, having a truncated-cone-shaped convex part protruding in a direction in which the liquid is atomized, and having multiple micropores in at least an upper base of the truncated-cone-shaped convex part, the multiple micropores penetrating the vibrating plate in a thickness direction, the liquid being supplied to the vibrating plate via the liquid absorbent wick.

Therefore, an ultrasonic wave atomizing device according to the present invention can improve diffusibility of an atomized liquid.

### Brief Description of Drawings

Fig. 1 is views schematically illustrating a vibrating plate according to this embodiment, and (a) of Fig. 1 is a top view and (b) of Fig. 1 is a cross-sectional view.
Fig. 2 is a view schematically illustrating an ultrasonic wave atomizing device according to this embodiment.
Fig. 3 is an enlarged view illustrating an atomizing section of an ultrasonic wave atomizing device according to this embodiment.
Fig. 4 is a view illustrating a modified example of an ultrasonic wave atomizing device according to this embodiment.
Fig. 5 is a view schematically illustrating another vibrating plate according to this embodiment (Reference), and (a) of Fig. 5 is a top view and (b) of Fig. 5 is a cross-sectional view.
Fig. 6 is a view schematically illustrating still another vibrating plate according to this embodiment (Reference), and (a) of Fig. 6 is a top view and (b) of Fig. 6 is a cross-sectional view.
Fig. 7 is an enlarged view illustrating another atomizing section of an ultrasonic wave atomizing device according to this embodiment.

### Description of Embodiments

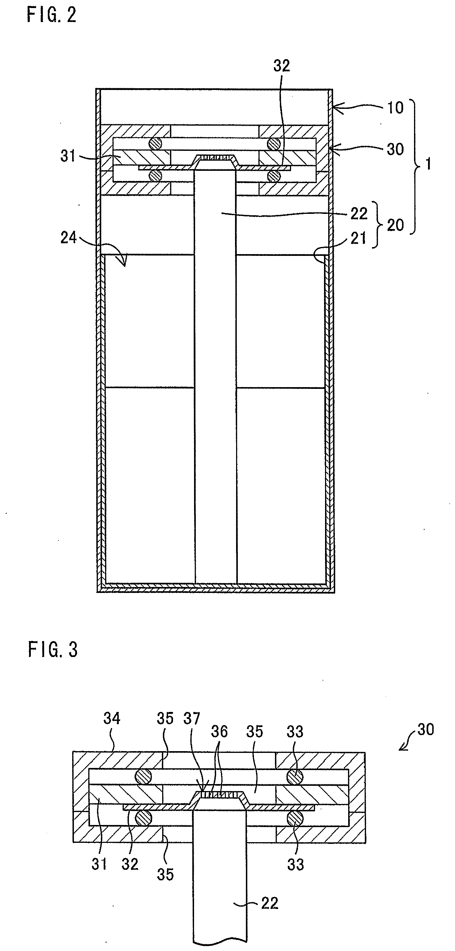

First, the following description discusses, with reference to Fig. 2 etc., an ultrasonic atomizing device 1 in accordance with this embodiment. Fig. 2 is a view schematically illustrating the ultrasonic atomizing device 1. Fig. 3 is an enlarged view illustrating an atomizing section 30 of the ultrasonic atomizing device 1.

### (Ultrasonic atomizing device 1)

The ultrasonic atomizing device 1 is a device for atomizing liquid such as water or a solution by ultrasonic vibration. The ultrasonic atomizing device 1 includes (i) a device body 10 which includes the atomizing section 30 and (ii) a solution container (liquid storage container) 20 which is housed in the device body 10. The following description is based on the assumption that the liquid is water or a solution such as insecticide, pesticide, or perfume.

### (Device body 10)

The device body 10 houses the solution container 20 therein and includes the atomizing section 30. The solution container 20 may be removably housed in the device body 10. As shown in Fig. 3, the atomizing section 30 includes (i) a piezoelectric element 31 which generates ultrasonic vibration when supplied with electricity, (ii) a vibrating plate 32 which atomizes a solution by vibration of the piezoelectric element 31, (iii) a couple of elastic rings 33 which are elastic annular members provided along a top surface of the piezoelectric element 31 and a bottom surface of the vibrating plate 32, respectively, and (iv) a casing 34 which holds the piezoelectric element 31 and the vibrating plate 32 by elastically sandwiching the piezoelectric element 31 and the vibrating plate 32 via the couple of elastic rings 33.

The piezoelectric element 31 is constituted by a thin circular piezoelectric ceramic plate which has an opening 35 at its center. The piezoelectric element 31 is polarized along its thickness direction, and generates ultrasonic vibration in a radial direction upon application of a high frequency voltage to electrodes (not illustrated) provided on both surfaces of the piezoelectric element 31. The piezoelectric element 31 is not limited provided that, for example, its thickness is 0.1 mm to 4.0 mm, its outer diameter is 6 mm to 60 mm. The piezoelectric element 31 has a ring shape when seen from a plan view, and has an opening in its center. The piezoelectric element 31 may have an oscillatory frequency of 30 kHz to 500 kHz.

The vibrating plate 32 is constituted by a thin circular plate made of nickel, nickel alloy, or ferroalloy. The vibrating plate 32 covers the opening 35 of the piezoelectric element 31, and, in Fig. 2, is joined (fastened) to a bottom surface of the piezoelectric element 31 so as to be concentric or substantially concentric (to substantially match) with the piezoelectric element 31. A thickness of the vibrating plate 32 is, for example, 0.02 mm to 2.0 mm, and an outer diameter of the vibrating plate 32 is, for example, 6 mm to 60 mm. The outer diameter of the vibrating plate 32 is selected as appropriate depending on the size of the piezoelectric element 31 so as to be larger than the inner diameter of the opening 35 of the piezoelectric element 31.

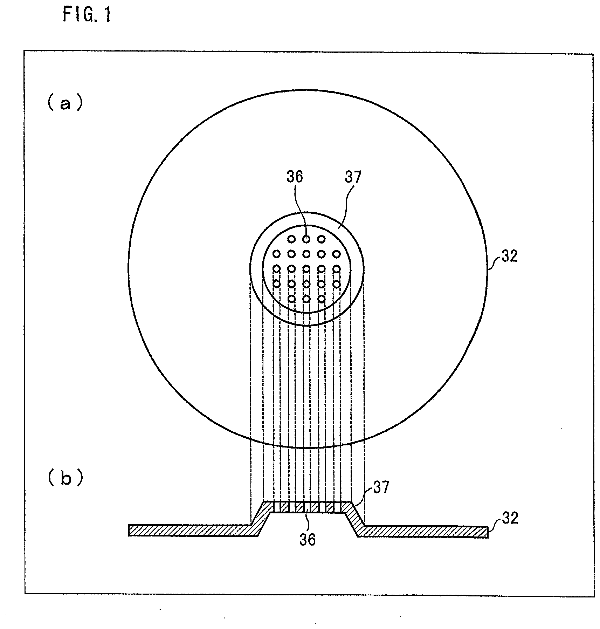

The vibrating plate 32 has, in its part that faces the opening 35 of the piezoelectric element 31, many micropores 36 passing through the vibrating plate 32 in a thickness direction. The diameter of each of the micropores 36 is preferably 3 µm to 150 µm. The vibrating plate will be described later with reference to Fig. 1 etc.

Note that, in Fig. 2, Fig. 3, and Fig. 4 described later, the micropores 36 are provided only in an upper base of the convex part 37 of the vibrating plate 32. However, it is herein disclosed that in the vibrating plate, the micropores 36 may be provided over a whole surface of the vibrating plate.

In the ultrasonic atomizing device 1, a solution is supplied to the micropores 36, the piezoelectric element 31 generates ultrasonic vibration when supplied with electricity, and ultrasonic vibration generated in the vibrating plate 32 by vibration of the piezoelectric element allows the solution to be atomized.

There is provided the couple of elastic rings 33. The couple of elastic rings 33 are in contact with the top surface of the piezoelectric element 31 and the bottom surface of the vibrating plate 32, respectively, so as to be concentric with the piezoelectric element 31 and the vibrating plate 32, respectively. Here, the couple of elastic rings 33 are in a state of elastic deformation between the casing 34 and the top surface of the piezoelectric element 31 and between the casing 34 and the bottom surface of the vibrating plate 32, respectively.

Each of the couple of elastic rings 33 is preferably an O-ring having a section diameter of 0.5 mm to 3 mm. Further, the hardness of the couple of elastic rings 33 is preferably 20 IRHD to 90 IRHD. Such a couple of elastic rings 33 makes it possible to hold the piezoelectric element 31 and the vibrating plate 32 with appropriate elasticity, and to hold the piezoelectric element 31 at a prescribed position within the casing 34 without restricting vibration of the piezoelectric element 31. Accordingly, it is possible to atomize a solution in a more stable manner.

It should be noted that an elastic ring 33 in contact with the top surface of the piezoelectric element 31 and an elastic ring 33 in contact with the bottom surface of the vibrating plate 32 are preferably the same in terms of mean diameter [(Inner diameter + Outer diameter) / 2], section diameter, and hardness etc. In particular, the couple of elastic rings 33 preferably have the same mean diameter.

The couple of elastic rings 33 are made from, for example, nitrile rubber, fluororubber, ethylene propylene rubber, silicone rubber, acrylic rubber, hydrogenated nitrile rubber, and/or the like.

Each of the couple of elastic rings 33 can be, instead of the O-ring, a ring that has, for example, an elliptic, rectangular, triangular, or rhombic cross section. Alternatively, each of the couple of elastic rings 33 can be a ring that has, for example, a D-shaped, X-shaped, or T-shaped cross section. Each of the couple of elastic rings 33 does not necessarily have to be a circumferentially continuous and complete ring, and therefore can have a slit in a circumferential direction or have several slits intermittently along the circumferential direction. Although the above description has discussed the vibrating plate 32 in the form of a thin circular plate which completely covers the opening 35 of the piezoelectric element 31, it is also possible to employ a vibrating plate in the form of a thin rectangular plate (i) which traverses the opening 35 of the piezoelectric element 31 and (ii) whose both ends are fastened to one surface of the piezoelectric element 31.

The atomizing section 30 is not limited to the above configuration, and can be constituted by a known piezoelectric atomizing section. The atomizing section 30 can be selected as appropriate.

### (Solution container 20)

The solution container 20 is constituted by a container body 21 and a liquid absorbent wick 22, and is removably housed in the device body 10.

The container body 21 is constituted by, for example, a cylindrical container which has a bottom surface and has an opening 24 at the top. The container body 21 contains a solution. The container body 21 is made from, for example, glass or synthetic resin.

The liquid absorbent wick 22 is, for example, made of nonwoven fabric and in columnar shape having a diameter of 2 mm to 6 mm. A lower portion of the liquid absorbent wick 22 is immersed in the solution contained in the container body 21. This makes it possible to supply the solution to an upper portion of the liquid absorbent wick 22 by capillary action.

The shape of the liquid absorbent wick 22 is not limited to a circular column, and can be a square column. The shape of the liquid absorbent wick 22 can be any shape. Furthermore, the thickness of the liquid absorbent wick 22 may be the thickness which allows the liquid absorbent wick 22 to pass through the opening 35 of the piezoelectric element 31 or through the inside of the convex part 37 of the vibrating plate 32.

The liquid absorbent wick 22 is preferably made of, for example, a porous material having continuous holes, a resin article having continuous cells, or an aggregation of resin fibers. Specific examples of materials from which the liquid absorbent wick 22 is made include, but not limited to: a resin article having continuous cells made of polyurethane, polyethylene, polyethylene terephthalate, polyvinyl formal, and polystyrene etc.; porous materials obtained by sintering of fine resin particles made mainly of polyethylene, polypropylene, and nylon, etc.; porous materials made of polyethylene fluoride etc.; aggregations of resin fibers, such as felt made of polyester, polypropylene, nylon, acrylic, rayon, wool, etc., and nonwoven fabric made of polyolefin fibers, polyester fibers, nylon fibers, rayon fibers, acrylic fibers, vinylon fibers, polychlal fibers, aramid fibers etc.; and porous sintered inorganic materials obtained by sintering of mainly inorganic powder such as ceramics. The specific examples of the materials further include the above materials treated with a surfactant.

How to house the solution container 20 in the device body 10 is not particularly limited, provided that (i) the solution container 20 is removably housed in the device body 10 and, (ii) while the device body 10 houses the solution container 20 therein, the liquid absorbent wick 22 is near or in contact with the convex part 37 (described later) of the vibrating plate 32. For example, the solution container 20 can be housed in the device body 10 by (i) being slidingly fitted into the device body 10 by being slid laterally or (ii) being rotatingly fitted into the device body 10 by being rotated laterally with a slight rotational angle.

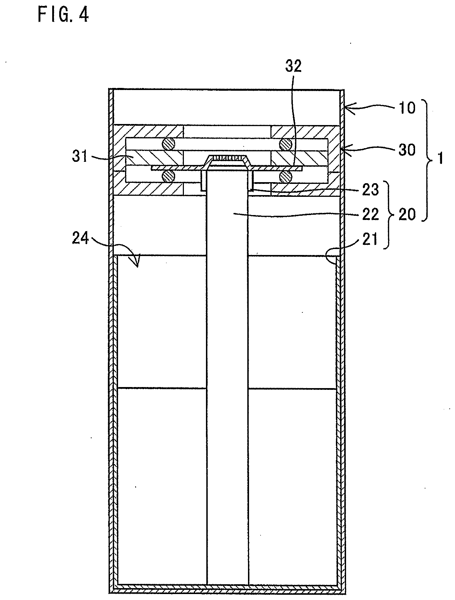

Supply of the solution to the vibrating plate 32 may be performed as follows, and this will be described with reference to Fig. 4. Fig. 4 is a view illustrating a modified example of an ultrasonic wave atomizing device according to this embodiment. Note that the matters which have been described already with reference to Fig. 2 etc. will be omitted.

As shown in Fig. 4, the solution container 20 includes the container body 21, the liquid absorbent wick 22, and the absorber 23, and is removably housed in the device body 10.

The liquid absorbent wick 22 is, for example, made of nonwoven fabric and in columnar shape having a diameter of 2 mm to 6 mm. A lower portion of the liquid absorbent wick 22 is immersed in the solution contained in the container body 21. This makes it possible to supply the solution to an upper portion of the liquid absorbent wick 22 by capillary action. The absorber 23 is provided to the upper portion of the liquid absorbent wick 22.

The absorber 23 is provided to the upper portion of the liquid absorbent wick 22 so as to be integral with the liquid absorbent wick 22. That is, when the solution container 20 is provided to or removed from the ultrasonic atomizing device 1, the absorber 23 is also provided to or removed from the ultrasonic atomizing device 1 together with the solution container 20. The absorber 23 lies near or is in contact with the convex part 3 7 of the vibrating plate 32, and supplies, to the convex part 37, the solution absorbed by the liquid absorbent wick 22. A material of the absorber 23 may be the same as that of the liquid absorbent wick 22.

Note that, in this embodiment, the term "integral" means (i) members constitute a single member, (ii) members are assembled together, or (iii) the like.

The absorbent wick 22 and the absorber 23 are fixed to the container body 21, and removably attached to the solution container 20 (or the container body 21).

According to the above structure, in a case where the solution container 20 is detached from the ultrasonic atomizing device 1, the absorber 23 is detached together with the solution container 20 from the ultrasonic atomizing device 1 to an outside thereof, and does not remain in the ultrasonic atomizing device 1. Therefore, in a case where the liquid in the solution container 20 is exhausted and the absorber 23 is dried, the absorber 23 as well as the solution container 20 is exchanged when the solution container 20 is exchanged, so that, when the ultrasonic atomizing device 1 is operated again, it is possible to prevent fibers etc. derived from the absorber 23 from clogging micropores of the vibrating plate 32. This effect is exerted particularly when the vibrating plate 32 is fixed to the side of the ultrasonic atomizing device 1.

Therefore, the ultrasonic atomizing device 1 can reduce the following situations: an atomizing amount of the liquid is unstable because of this clogging; and a user is forced to exchange a high-cost vibrating plate.

The liquid absorbent wick 22 and/or the absorber 23 may be structured to be fixed to the side of the ultrasonic atomizing device 1.

### (Vibrating plate)

Next, the following description will discuss the vibrating plate 32 in detail with reference to Fig. 1. Fig. 1 is views schematically illustrating the vibrating plate 32, and (a) of Fig. 1 is a top view and (b) of Fig. 1 is a cross-sectional view.

As described above, the vibrating plate 32 is a circular thin plate made from nickel, nickel alloy, or ferroalloy and is joined (fastened) to the bottom surface of the piezoelectric element 31 so as to be concentric or to be substantially concentric (to substantially match) with the piezoelectric element 31. The vibrating plate 32 includes a convex part 37 protruding to have a truncated-cone shape, and the convex part 37 is provided so that the truncated-cone shape may be concentric with the piezoelectric element 31. As shown in (a) of Fig. 1 and (b) of Fig. 1, the plurality of micropores 36 are provided only in an upper base of the convex part 37.

Here, a frustum refers to a solid figure which is obtained by removing, from a cone or pyramid, a cone or pyramid which has the same apex and is reduced in size similarly. In other words, the frustum is a solid figure surrounded by a conical/pyramid surface and two parallel planes. In this embodiment, a frustum having a cone shape is referred to as "truncated cone", a frustum having a pyramid is referred to as "truncated pyramid", and a frustum having an n-gonal pyramid is referred to as "n-gonal truncated pyramid".
As described above, according to the invention, the convex part 37 has a truncated-cone shape when an upper surface on which the plurality of micropores 36 are provided is considered as the upper base and a rising surface of the convex part 37 in the vibrating plate 32 is considered as the conical surface.
In addition, herein is disclosed that the convex part 37 has a truncated-cone shape when an upper surface on which the plurality of micropores 36 are provided is considered as the upper base and a rising surface of the convex part 37 in the vibrating plate 32 is considered as the pyramid surface. In addition, the liquid absorbent wick 22 and the absorber 23 are located in a nonexistent part corresponding to a lower base, and the liquid is supplied to the convex part 37 from the absorber 23.

More specifically, the following description will discuss a case where a frustum has a truncated-cone shape. The upper base of the truncated-cone-shaped convex part 37 preferably has a diameter smaller than that of the cylindrical liquid absorbent wick 22. Although not existed, the lower base of the convex part 37 preferably has a diameter which is the same as or slightly larger than that of the liquid absorbent wick 22. In addition, the upper base of the truncated-cone-shaped convex part 37 preferably has a diameter of 1.0 mm or more but 7.0 mm or less. The lower base of the convex part 37 preferably has a diameter of 2.2 mm or more but 11.0 mm or less. The convex part 37 preferably has a height (distance between upper base and lower base) of 0.1 mm or more but 2.0 mm or less. An angle between the lower base of the convex part 37 and a slanting surface of the convex part 37 is preferably 45° or less.

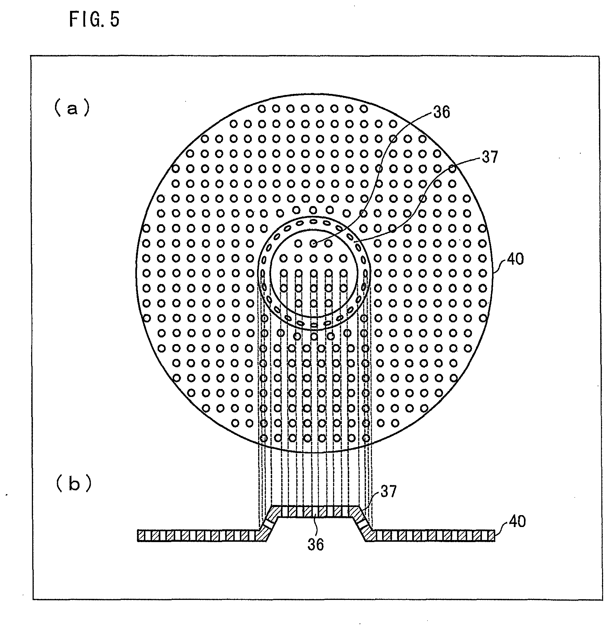

Fig. 5 illustrates a modification example of a vibrating plate according to this embodiment. Fig. 5 is a view schematically illustrating a vibrating plate 40, and (a) of Fig. 5 is a top view and (b) of Fig. 5 is a cross-sectional view.

The vibrating plate 40 is different from the vibrating plate 32 in the following point. That is, in the vibrating plate 32, the micropores 36 are provided only in the upper base of the convex part 37 of the vibrating plate 32, however, in the vibrating plate 40, the micropores 36 are provided in the whole vibrating plate 40. According to the invention, the vibrating plate 32 can be used. In addition, herein disclosed is that the vibrating plate 40 can be used.

Note that effect brought about by the vibrating plate 32 and the vibrating plate 40 will be described below with an effect confirmation test described later.

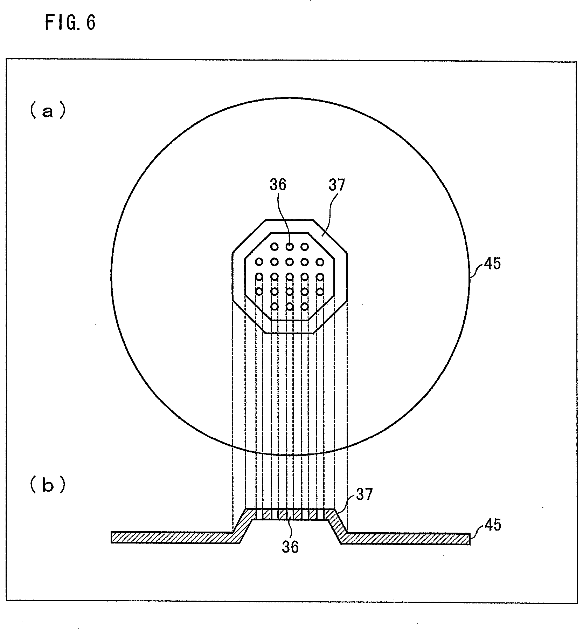

Fig. 6 illustrates a modified example (Reference) of a vibrating plate according to this embodiment. Fig. 6 is a view schematically illustrating a vibrating plate 45, and (a) of Fig. 6 is a top view and (b) of Fig. 6 is a cross-sectional view.

The vibrating plate 45 is different from the vibrating plate 32 in the following point. That is, in the vibrating plate 32, the convex part 37 has a truncated-cone shape, whereas, in the vibrating plate 45, the convex part 37 has an octagonal frustum shape. In this embodiment, not only the vibrating plate 32 and/or the like but also the vibrating plate 45 can be used.

In Fig. 6, the plurality of micropores 36 are provided only in the upper base of the convex part 37 of the vibrating plate 45. However, the vibrating plate 45 may be structured as described above, in addition, may be structured in which the plurality of micropores 36 are provided not only in the upper base but also in a side surface section of the convex part 37, or are provided in the whole vibrating plate 45 including the side surface section. In the above description, the vibrating plate 45 has been described as a plate having an octagonal frustum shape. However, the vibrating plate 45 may have an n-gonal frustum shape such as a quadrangular frustum shape, a hexadecagonal frustum shape, or the like.

All the vibrating plate 32, the vibrating plate 40, and the vibrating plate 45 are the same in that (i) a cross section thereof in a direction in which the solution is to be atomized has a trapezoid shape and (ii) a surface having an atomizing opening of the solution is a plane surface. Note, however, that upper bases and side surfaces of the frustum-shaped vibrating plate 32, the vibrating plate 40, and the vibrating plate 45 do not need to be exactly plane, and may be a surface having a slight curvature.

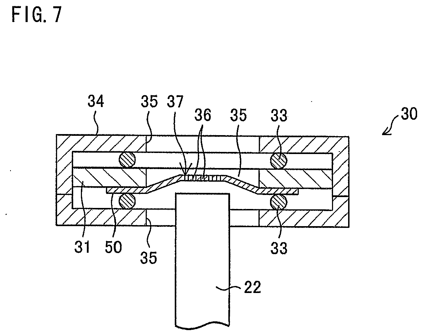

In the atomizing section 30 shown in Fig. 3, a rising section of the truncated-cone-shaped convex part 37 provided on the vibrating plate 32 is close to a center, however, as shown in Fig. 7, there may be used the vibrating plate 50 in which the rising section of the truncated-cone-shaped convex part 37 is close to an inner peripheral surface of the piezoelectric element 31.

### (Effect Confirmation Test)

The following description will discuss an ultrasonic wave atomizing device according to this embodiment more specifically with reference to the following examples. Note, however, that an ultrasonic wave atomizing device according to this embodiment is not limited thereto.

### (Production of ultrasonic atomizing device)

An ultrasonic atomizing device having the following specifications was produced.
(1) Piezoelectric element 31: Piezoelectric ceramics whose outer diameter is 15 mm, inner diameter is 5 mm, and thickness is 0.4 mm
(2) Applied voltage: 30 Vp-p
(4) Frequency of piezoelectric element 31 (ultrasonic exciter): 110 kHz

### (Example)

As Example, the inventive vibrating plate 32 and the disclosed vibrating plate 40 each made from nickel were used. The micropores 36 are provided only in the upper base of the vibrating plate 32, whereas the micropores 36 are provided on the whole vibrating plate 40. The micropores of both the vibrating plates 32 and 40 have a diameter of 6.0 µm, and sizes of the vibrating plates 32 and 40 are such that a diameter of the upper base of the convex part is 2.5 mm, a diameter of the lower base of the convex part is 5.0 mm, and a height of the convex part is 0.2 mm. Note that a solution used in this example was ethanol (viscosity: 1.17 mPa·s (at 20 °C)). This solution was also used in the following Comparative Example.

### (Comparative Example)

As Comparative Example, a vibrating plate A and a vibrating plate B each made from nickel and having a dome shape were used. Here, the "dome shape" means a shape of a vibrating plate whose convex part is expanded in a direction in which a solution is atomized so as to have an R shape. The vibrating plate A has micropores only in a surface constituting a dome. The vibrating plate B has micropores in its whole surface. The micropores of the vibrating plates A and B have a diameter of 6 µm, and sizes of the vibrating plates A and B are such that a diameter of a base end section of the convex part is 3 mm and a height of the convex part is 0.2 mm.

The solution was atomized under the above conditions in Example and in Comparative Example, and a height from an atomizing opening to an observable farthest destination point of a mist was measured visually. Note that an average value of results of 10 measurements was employed as a measurement value.

As a result, the farthest destination point of the mist in (Example) was 41.3 cm in a case of using the vibrating plate 32, and was 33.1 cm in a case of using the vibrating plate 40. Meanwhile, the farthest destination point of the mist in (Comparative Example) was 24.3 cm in a case of using the vibrating plate A and was 23.8 cm in a case of using the vibrating plate B.

That is, the results showed that the farthest destination point of the mist was higher in a case of the truncated-cone-shaped vibrating plate 32 and the truncated-cone-shaped vibrating plate 40 according to Example than in a case of the dome-shaped vibrating plate A and the dome-shaped vibrating plate B in Comparative Example. A comparison of the numerical values revealed that a solution atomizing height of the vibrating plate 32 was about 1.7 times as high as those of the vibrating plate A and the vibrating plate B, and a solution atomizing height of the vibrating plate 40 was about 1.4 times as high as those of the vibrating plate A and vibrating plate B.

From this above, Example demonstrated that diffusibility of the solution was improved by increasing the atomizing height and, as a result, effects (humidification effect, fragrance effect, insecticidal and sterilizing effect, etc.) of the solution were widely spread and brought about immediately, as compared with Comparative Example. Therefore, Example is effective particularly when the present invention is used as an insecticide or the like which needs to have an immediate effect.

Furthermore, Example had the inventors of the present invention find that durability of vibrating plates was improved.

The following description will discuss Example and Comparative Example specifically. The vibrating plates used in the above (Example) and (Comparative Example) were used and were driven under a driving condition that a device continuously performed idle driving at an interval of 2 seconds ON and 8 seconds OFF for 20 hours, and a degradation ratio of the vibrating plates were compared. Here, degradation of the vibrating plates indicates a case where 50% or more of an atomizing amount is reduced before and after this experiment.

As a result of 9 experiments, in Example, none of the vibrating plate 32 was degraded. That is, the degradation ratio was 0%. Further, 2 vibrating plates 40 were degraded in 9 experiments. That is, the degradation ratio was 2/9 = 22%.

Meanwhile, in Comparative Example, 3 vibrating plates A and 3 vibrating plates B were degraded in 5 experiments. That is, each of the vibrating plates A and B had a degradation ratio of 60%.

The result showed that the degradation ratios of the truncated-cone-shaped vibrating plates according to Example were lower than those of dome-shaped vibrating plates according to Comparative Example. Particularly, the vibrating plate 32 having the micropores 36 only in the upper base has the degradation ratio of 0%, meanwhile, the degradation ratio was 60% in Comparative Example, so that the durability of the vibrating plate 32 is extremely high.

The truncated-cone-shaped convex part 37 of the vibrating plate 32 and a dome part of the vibrating plate A (B) are both formed by subjecting a plane vibration plate having micropores to a press process. In the vibrating plate A (B), when the plane vibration plate is pressed, process distortion occurs in the whole dome part, in particular, in a top portion of the dome part. On the contrary, process distortion rarely occurs in the upper base of the truncated-cone-shaped convex part 37 of the vibrating plate 32 (40). Therefore, it is considered that a crack is generated by vibration at the time of driving more easily in the top part of the dome part than in the upper base of the truncated-cone-shaped convex part 37, and the degradation ratio of the vibrating plate is higher.

It is quite possible that a user unknowingly turns on the device in a state in which the solution container is not provided or the solution is exhausted (solution container becomes empty) while the device is being used, and, in such a case, if the degradation of the vibrating plate can be reduced, a life of the device can be prolonged. That is, by improving the durability of the device, it is possible to reduce a burden on a user also in terms of costs.

As described above, the farthest destination points of the mist in the vibrating plates according to Example are higher than those in the vibrating plates according to Comparative Example. This makes it possible to improve diffusibility of the solution. In addition, by widely spreading effects (humidification effect, fragrance effect, insecticidal and sterilizing effect, etc.) of the solution, a high immediate effectiveness is expected.

The degradation ratios of the vibrating plates according to Example can be remarkably reduced in comparison with the vibrating plates according to Comparative Example. Therefore, as long as the vibrating plates are made from a same material, it is possible to prolong the life of the device by using the vibrating plates according to Example, which results in reduction of a frequency of exchanging the device and a burden on a user is reduced in terms of costs.

### (Supplementary remarks)

In an ultrasonic wave atomizing device according to the present invention, the multiple micropores are provided only in an upper base of the convex part of the vibrating plate.

According to the above structure, the ultrasonic wave atomizing device according to the present invention further improves an atomizing height of a liquid and durability of the vibrating plate.

In addition, herein disclosed is that in the ultrasonic wave atomizing device, the multiple micropores may be provided over a whole area of the vibrating plate.

According to the above structure, the ultrasonic wave atomizing device according to the present invention can further increase the atomizing height of the liquid and the durability of the vibrating plate, as compared with a conventional ultrasonic wave atomizing device including a dome-type vibrating plate.

In the ultrasonic wave atomizing device according to the present invention, the convex part of the vibrating plate has
a truncated-cone shape.
In addition, herein disclosed is that in the ultrasonic wave atomizing device,
the convex part of the vibrating plate may have a truncated-pyramid shape.
In addition, herein disclosed is that in the ultrasonic wave atomizing device,
the convex part of the vibrating plate may have a truncated-cone shape or a truncated-pyramid shape.

Therefore, in each device, in order to match the shape of the convex part of the vibrating plate with a layout of the device or a shape of the liquid absorbent wick, the shape may be changed to a truncated-cone shape or truncated-pyramid shape, so that a design of the device can be changed flexibly.

The ultrasonic wave atomizing device according to the present invention may be structured such that: the liquid storage container is removably housed in the ultrasonic wave atomizing device; the liquid storage container includes an absorber for supplying, to the vibrating plate, the liquid absorbed by the liquid absorbent wick; and the absorber is configured to be provided to or removed from the ultrasonic atomizing device together with the liquid storage container when the liquid storage container is provided to or removed from the ultrasonic atomizing device.

According to the above structure, in a case where the liquid storage container is detached from the ultrasonic atomizing device, the absorber is detached together with the liquid storage container from the device to an outside thereof, and does not remain in the ultrasonic atomizing device. Therefore, in a case where the liquid in the liquid storage container is exhausted and the absorber is dried, the absorber as well as the liquid storage container is exchanged when the liquid storage container is exchanged, so that, when the ultrasonic atomizing device is operated again, it is possible to prevent fibers etc. derived from the absorber from clogging micropores of the vibrating plate. This effect is exerted particularly when the vibrating plate is fixed to the side of the device body.

Therefore, the ultrasonic atomizing device according to the present invention can reduce the following situations: an atomizing amount of the liquid is unstable because of this clogging; and a user is forced to exchange a high-cost vibrating plate.

As described above, because the ultrasonic wave atomizing device according to the present invention has the above structure, it is possible to reduce a burden on a user in terms of costs, and, by reducing clogging of the micropores of the vibrating plate, it is possible to improve atomizing stability of the ultrasonic wave atomizing device.

In the ultrasonic wave atomizing device according to the present invention, the truncated-cone-shaped convex part may have a rising section provided to be close to an inner peripheral surface of the piezoelectric element.

Various forms of the ultrasonic wave atomizing device according to this embodiment have been described above. Those forms are an example of this embodiment, and, as a matter of course, the forms described herein can be combined.

The present invention is not limited to the description of the embodiments above, and can be modified in numerous ways by a skilled person as long as such modification falls within the scope of the claims. An embodiment derived from a proper combination of technical means disclosed in different embodiments is also encompassed in the technical scope of the present invention.

### Industrial Applicability

The present invention is suitably used as an ultrasonic wave atomizing device for humidification, fragrance, sterilization and killing insects.

### Reference Signs List

- 1: ultrasonic wave atomizing device
- 10: device body
- 20: solution container (liquid storage container)
- 21: container body
- 22: liquid absorbent wick
- 23: absorber
- 24: opening
- 30: atomizing section
- 31: piezoelectric element
- 32, 40, 45, 50: vibrating plate
- 33: elastic rings
- 34: casing
- 35: opening
- 36: micropores
- 37: the convex part

## Claims

1. An ultrasonic wave atomizing device (1) for liquid atomization, comprising:
a liquid absorbent wick (22) for absorbing a liquid from a liquid storage container (20); and
a vibrating plate (32, 50) which has multiple micropores (36) penetrating the vibrating plate (32, 50) in a thickness direction and atomizes the liquid by vibration of a piezoelectric element (31) which generates ultrasonic vibration when supplied with electricity,
the vibrating plate (32, 50) having a convex part (37) which has a truncated-cone shape and is supplied with the liquid through the liquid absorbent wick (22),
wherein the multiple micropores (36) are provided only in an upper base of the convex part (37) of the vibrating plate (32, 50), and
wherein the vibrating plate (32, 50) is constituted by a thin circular plate made of nickel, nickel alloy, or ferroalloy.

2. The ultrasonic wave atomizing device (1) according to claim 1, wherein:
the liquid storage container (20) is removably housed in the ultrasonic wave atomizing device (1);
wherein the liquid storage container (20) includes an absorber (23) for supplying, to the vibrating plate (32, 50), the liquid absorbed by the liquid absorbent wick (22); and
the absorber (23) is configured to be provided to or removed from the ultrasonic wave atomizing device (1) together with the liquid storage container (20) when the liquid storage container (20) is provided to or removed from the ultrasonic wave atomizing device (1).

## Patentansprüche

1. Ein Ultraschallwellenzerstäuber (1) zur Flüssigkeitszerstäubung, umfassend:
einen flüssigkeitsabsorbierenden Docht (22) zum Absorbieren einer Flüssigkeit aus einem Flüssigkeitsspeicherbehälter (20); und
eine Vibrationsplatte (32, 50), die mehrere Mikroporen (36) aufweist, die die Vibrationsplatte (32, 50) in einer Dickenrichtung durchdringen und die Flüssigkeit durch Vibration eines piezoelektrischen Elements (31), welches Ultraschallschwingungen erzeugt, wenn es mit Elektrizität versorgt wird, zerstäuben,
wobei die Vibrationsplatte (32, 50) einen konvexen Teil (37) mit einer Kegelstumpfform aufweist und durch den flüssigkeitsabsorbierenden Docht (22) mit der Flüssigkeit versorgt wird,
wobei die mehrfachen Mikroporen (36) nur in einer oberen Basis des konvexen Teils (37) der Vibrationsplatte (32, 50) bereitgestellt sind, und
wobei die Vibrationsplatte (32, 50) aus einer dünnen kreisförmigen Platte aus Nickel, einer Nickellegierung oder einer Ferrolegierung gebildet ist.

2. Der Ultraschallwellenzerstäuber (1) nach Anspruch 1, wobei:
der Flüssigkeitsspeicherbehälter (20) abnehmbar in dem Ultraschallwellenzerstäuber (1) untergebracht ist;
wobei der Flüssigkeitsspeicherbehälter (20) einen Absorber (23) beinhaltet, um der Vibrationsplatte (32, 50) die vom flüssigkeitsabsorbierenden Docht (22) absorbierte Flüssigkeit zuzuführen; und
der Absorber (23) so konfiguriert ist, dass er zusammen mit dem Flüssigkeitsspeicherbehälter (20) dem Ultraschallwellenzerstäuber (1) zugeführt oder von ihm entfernt wird, wenn der Flüssigkeitsspeicherbehälter (20) dem Ultraschallwellenzerstäuber (1) zugeführt oder von ihm entfernt wird.

## Revendications

1. Dispositif d'atomisation à ondes ultrasoniques (1) pour l'atomisation d'un liquide, comprenant :
une mèche absorbant les liquides (22) pour l'absorption d'un liquide à partir d'un récipient de stockage de liquide (20) ; et
une plaque vibrante (32, 50) qui a de multiples micropores (36) pénétrant dans la plaque vibrante (32, 50) dans la direction de l'épaisseur et atomise le liquide par vibration d'un élément piézoélectrique (31) qui génère une vibration ultrasonique lorsqu'elle est alimentée en électricité,
la plaque vibrante (32, 50) ayant une partie convexe (37) qui a une forme de cône tronqué et est alimentée par le liquide par l'intermédiaire de la mèche absorbant les liquides (22),
dans lequel les multiples micropores (36) sont disposés uniquement dans une base supérieure de la partie convexe (37) de la plaque vibrante (32, 50), et
dans lequel la plaque vibrante (32, 50) est constituée d'une plaque circulaire mince faite en nickel, en un alliage de nickel, ou en un ferro-alliage.

2. Dispositif d'atomisation à ondes ultrasoniques (1) selon la revendication 1, dans lequel :
le récipient de stockage de liquide (20) est logé de façon amovible dans le dispositif d'atomisation à ondes ultrasoniques (1) ;
dans lequel le récipient de stockage de liquide (20) comprend un absorbeur (23) pour fournir, à la plaque vibrante (32, 50), le liquide absorbé par la mèche absorbant le liquide (22) ; et
l'absorbeur (23) est configuré de façon à être disposé dans ou retiré du dispositif d'atomisation à ondes ultrasoniques (1) conjointement avec le récipient de stockage de liquide (20) quand le récipient de stockage de liquide (20) est disposé dans ou retiré du dispositif d'atomisation à ondes ultrasoniques (1).
